# EUROPEAN PATENT APPLICATION

(11) **EP 4 603 471 A1**
(43) Date of publication of application: **20.08.2025**
(21) Application number: 24157848.3
(22) Date of filing: 15.02.2024
(51) Int. Cl.: C07C 27/04, C01B 21/12, B01D 53/00, B01D 53/58, C05C 9/00, C07C 273/16

(54) **METHOD FOR PRODUCING STEAM**

(71) Applicant: Yara International ASA, 0277 Oslo (NO)
(72) Inventor: Porro, Lino Giovanni, 1040 Etterbeek (BE); Dorini, Francesco, 44122 Ferrara (IT)
(74) Representative: De Clercq & Partners

(57) **Abstract**

The present disclosure provides a method for producing steam in a carbamate condenser, comprising the steps of: a) providing a gaseous stream comprising ammonia, carbon dioxide, and water to the carbamate condenser, b) providing an aqueous cooling solution to the cooling fluid inlet, obtaining a fluid composition comprising an aqueous solution comprising ammonium salts from the carbamate condenser, and obtaining steam from the cooling fluid outlet of the carbamate condenser. In another aspect, the present disclosure provides a method for producing a urea-containing composition.

## Description

### Field of the disclosure

The present disclosure is related to the field of chemical engineering, in particular urea production.

### Background information

Worldwide production of urea, with the chemical formula H₂NCONH₂, is estimated to be around 180 million metric tons. It is primarily used as a fertilizer to provide nitrogen to plants, but it can also be used in other production processes, or as an additive in diesel-driven cars to reduce greenhouse gas emissions.

Different production technologies are available today, but they all rely on a common design: ammonia and carbon dioxide are mixed in a synthesis reactor at high temperature (above 150 °C) and high pressure (above 10 MPa) to produce an aqueous urea solution that is processed to provide a urea melt having a urea concentration above 95%. A urea melt can be particulated into a solid, particulate composition with various techniques, such as prilling, and granulation, or it can be diluted in water to provide an aqueous urea solution.

During the processing of the solution produced by the synthesis reactor, gaseous streams comprising ammonia, carbon dioxide and water are produced. To increase the overall yield of the plant, these streams are sent back to the synthesis reactor in liquid form. The gaseous streams are directed to heat exchangers that also receive a cooling medium, for example water, which cools down the gaseous stream, such that it condenses into an aqueous solution comprising ammonium salts.

The heated cooling medium produced by the heat exchanger becomes a waste stream for the plant. In conventional plants, the heated cooling medium is cooled by ambient air or water in another heat exchanger, thereby releasing heat in the atmosphere, which is highly undesirable.

So, there is a need to provide a method that reduces the environmental footprint of a urea-producing plant.

### Summary of the disclosure

It was found that it is possible to recover the heat generated during the condensation of a gaseous stream comprising ammonia, carbon dioxide, and water, in a carbamate condenser operating at a pressure lower than 4.0 MPa such that the heat was not released in the atmosphere. The heat generated by the condensation can be used to produce steam, in particular low-pressure steam, which can be re-used in the urea-producing plant.

In a first aspect, the present disclosure provides a method for producing steam in a carbamate condenser, the carbamate condenser comprising a fluid inlet, a fluid outlet, a cooling fluid inlet, and a cooling fluid outlet, the method comprising the steps of:
a) providing a gaseous stream comprising ammonia, carbon dioxide, and water to the fluid inlet, wherein the gaseous stream has a temperature ranging from 100 to 140 °C and a pressure ranging from 0.1 to 4.0 MPa;
b) providing an aqueous cooling solution to the cooling fluid inlet, wherein the temperature of the aqueous cooling solution ranges from 60 to 110 °C;
c) obtaining a fluid composition from the fluid outlet, the fluid composition comprising an aqueous solution comprising ammonium salts;
d) obtaining steam from the cooling fluid outlet.

In another aspect, the present disclosure provides a method for producing a urea-containing composition, the method comprising the steps of:
a) reacting ammonia and carbon dioxide in a synthesis section, thereby producing a synthesis solution comprising urea, water, and ammonium carbamate;
b) processing the synthesis solution produced in step a), thereby producing a urea melt comprising urea and water, and one or more gaseous streams comprising ammonia, carbon dioxide, and water;
c) condensing at least one of the one or more gaseous streams in a carbamate condenser to produce steam, thereby producing a fluid composition comprising an aqueous solution comprising ammonium salts, and steam;
d) processing the urea melt produced in step b) into a urea-containing composition.

In another aspect, the present disclosure provides a urea-producing plant for producing a urea-based composition comprising:
- a synthesis section configured to produce an aqueous urea solution comprising urea, water, and ammonium carbamate;
- a decomposition section connected to the synthesis section, the decomposition section being configured to receive an aqueous urea solution, and transform said aqueous urea solution into a pure urea solution comprising urea, water, and less than 5.0 weight% of impurities;
- an evaporation section connected to the decomposition section configured to remove water from the pure urea solution comprising urea, water, and less than 5.0 weight% of impurities to obtain a urea melt comprising urea and less than 5.0 weight% of water;
- a finishing section configured to receive a urea melt and produce a urea-containing product;
- a condensation section connected to the decomposition section and the evaporation section, wherein:
- the condensation section is configured to transform gaseous streams comprising ammonia, carbon dioxide and water, from the decomposition section and/or the evaporation section, into an aqueous solution comprising ammonium salts;
- the condensation section is also connected to the synthesis section and configured to send the aqueous solution comprising ammonium salts to the synthesis section; and
- the condensation section comprises at least one carbamate condenser configured to produce low-pressure steam from the condensation of a gaseous stream comprising ammonia, carbon dioxide, and water.

In another aspect, the present disclosure provides the use of a carbamate condenser to produce steam, wherein the carbamate condenser receives a gaseous stream comprising ammonia, carbon dioxide, and water to the fluid inlet, the gaseous stream having a temperature ranging from 100 to 140 °C and a pressure ranging from 0.1 to 4.0 MPa.

### Brief description of the figures

The following description of the figure of a specific embodiment of a system according to the present disclosure is only given by way of example and is not intended to limit the present explanation, its application or use. In the figure, identical reference numerals refer to the same or similar parts and features.
Figure 1 shows a carbamate condenser that can perform the method described in the present application.
Figure 2 shows a schematic representation of a urea-producing plant wherein the method as described in the present disclosure can be performed.
Figure 3 shows a schematic representation of another urea-producing plant suitable for producing urea according to a method disclosed herein.

### Detailed description of the disclosure

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a device" refers to one or more than one device.
"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g., component and do not exclude or preclude the presence of additional, non-recited components, features, element, members, steps, known in the art or disclosed therein.
The recitation of numerical ranges by endpoints includes all numbers and fractions subsumed within that range, as well as the recited endpoints.
The expression "weight percent", "°/wt" or "weight%", here and throughout the description unless otherwise defined, refers to the relative weight of the respective component based on the overall weight of the formulation.

In a first aspect, the present disclosure provides a method for producing steam in a carbamate condenser, the carbamate condenser comprising a fluid inlet, a fluid outlet, a cooling fluid inlet, and a cooling fluid outlet, the method comprising the steps of:
a) providing a gaseous stream comprising ammonia, carbon dioxide, and water to the fluid inlet, wherein the gaseous stream has a temperature ranging from 100 to 140 °C and a pressure ranging from 0.1 to 4.0 MPa;
b) providing an aqueous cooling solution to the cooling fluid inlet, wherein the temperature of the aqueous cooling solution ranges from 60 to 110 °C;
c) obtaining a fluid composition from the fluid outlet, the fluid composition comprising an aqueous solution comprising ammonium salts;
d) obtaining steam from the cooling fluid outlet.

The condensation of gaseous stream comprising ammonia, carbon dioxide, and water, produces an aqueous solution comprising ammonium salts, such as ammonium carbonate, ammonium bicarbonate, and ammonium carbamate, and releases energy as heat, in particular from the enthalpy of condensation of the gaseous water and from the reaction of ammonia and carbon dioxide to form ammonium salts. When a gaseous stream comprising ammonia, carbon dioxide, and water, having a high pressure, at least 4.0 MPa, and/or a high temperature, is condensed in a heat exchanger, such as a shell-and-tube exchanger, it is possible to produce steam having a pressure of at least 0.3 MPa. In urea-producing plants, such heat exchanger is often named high-pressure carbamate condenser. That steam can be directed to other devices, such as a carbamate decomposer, in the urea plant that require steam to operate. Recovering the condensation and heat enthalpies via steam enables the plant to lower its overall energy consumption by decreasing its steam production.

However, a urea plant also produces gaseous streams comprising ammonia, carbon dioxide, and water, having a pressure below 4.0 MPa and/or a temperature below 140 °C. Conventionally, these streams are condensed in carbamate condensers, such as shell-and-tube carbamate condensers, using cooling water, thereby producing heated cooling water. This heated cooling water needs to be cooled down to be re-used in the plant. This is conventionally done in a cooling tower using cold water, which is then discharged in the environment, or in an air cooler using air as cooling medium. This cooling eventually leads to a discharge of heat in the environment which contributes to climate change and is undesirable.

It was found that it was possible to produce steam from the condensation of such low pressure and/or low temperature gaseous streams comprising ammonia, carbon dioxide, and water. The steam produced is re-used in the urea plant, and therefore not released in the atmosphere thereby lowering the environmental footprint of the plant. The steam produced is a low-pressure steam.

A gaseous stream comprising ammonia, carbon dioxide, and water, is provided to the fluid inlet of the carbamate condenser. The gaseous stream has a temperature ranging from 100 to 140 °C and a pressure ranging from 0.1 to 4.0 MPa. The gaseous stream is typically produced by a device within the urea plant, for example a carbamate decomposer. A carbamate decomposer is a device configured to receive an aqueous solution comprising urea and ammonium salts, such as ammonium carbamate, ammonium carbonate, and/or ammonium bicarbonate. In the carbamate decomposer, the aqueous solution is heated up, for example with steam. Heating the solution triggers the decomposition of ammonium salts, in particular ammonium carbamate, into free ammonia and free carbon dioxide, which evaporate from the aqueous solution. The carbamate decomposer produces an aqueous urea solution depleted of ammonium ions and a gaseous stream comprising ammonia, carbon dioxide, and water. Urea plants also comprise evaporators to remove water from aqueous urea solutions. An evaporator is configured to receive an aqueous solution comprising urea and water and to remove some of the water comprised in the aqueous solution. Removing water is performed by heating the solution, which causes some of the urea comprised in the solution to decompose into ammonia and carbon dioxide. An evaporator produces a concentrated urea solution or urea melt, and a gaseous stream comprising ammonia, carbon dioxide, and water.

An aqueous cooling solution is provided to the cooling fluid inlet, wherein the temperature of the aqueous cooling solution ranges from 60 to 110 °C. The aqueous cooling solution may comprise at least 99.0 wt%, at least 99.5 weight%, or at least 99.9 weight% of water. In some embodiments, the aqueous cooling solution is 100% water. The aqueous cooling solution is partially or fully transformed in steam in the carbamate condenser, so it should be very pure. The aqueous cooling solution should have a temperature of at least 60 °C. Such temperature is required for two reasons: first, when the gaseous stream comprising ammonia, carbon dioxide, and water, condenses, an aqueous solution comprising ammonium salts is formed. Some ammonium salts, such as ammonium carbamate, have a poor water solubility and can crystallize at temperatures around 20 °C, so it is important to maintain the condensed liquid at a temperature of at least 60 °C. Secondly, the objective of the method is to produce steam from the aqueous cooling solution. Having a cooling aqueous solution at high temperature facilitates the production of steam since less heat is wasted to heating up the aqueous cooling solution to its boiling point.

The cooling fluid inlet and cooling fluid outlet are connected to each other via a cooling network.

As the gaseous stream comprising ammonia, carbon dioxide, and water travels in the carbamate condenser, it comes into contact with a cooler surface and the water comprised in the gaseous stream begins to condensate into liquid water. As liquid water is formed, the gaseous ammonia and carbon dioxide comprised in the gaseous stream transfer into the liquid phase and react together to form ammonium salts, such as ammonium carbamate, ammonium carbonate, and ammonium bicarbonate. The liquid condensate may contain one or more of these salts. The ratios of the different ammonium salts depend on different factors, such as the content in ammonia and carbon dioxide of the gaseous stream, the pressure and temperature of the condensate. The reaction between the free ammonia and free carbon dioxide solubilized in the condensate drives the solubilization of the gaseous compounds into the condensate.

As the gaseous stream comprising ammonia, carbon dioxide, and water, condensates into an aqueous solution, heat is released and absorbed by the cooling aqueous solution. If the aqueous cooling solution is at its boiling point, water evaporates and steam is obtained. The steam exits the carbamate condenser via the cooling fluid outlet.

In some embodiments, the steam obtained from the cooling fluid outlet has a pressure ranging from 20 to 150 kPa. Such steam is considered low-pressure steam in a urea-producing plant.

In some embodiments, the pressure inside the cooling network is ranging from 20 to 150 kPa. It may be an advantage that the aqueous cooling solution provided to the heat exchanger has a temperature which is from 0 to 10 °C, from 0 to 5 °C, or from 0 to 2°C lower than its boiling temperature. If the temperature of the aqueous cooling solution is close to its boiling point, most of the energy released by the condensation is used to boil water into steam. If the temperature of the aqueous cooling solution is much lower than its boiling point, part of the energy released by the condensation is used to heat up water to its boiling point, thereby lowering the amount of steam produced. The boiling point of water is dependent on its pressure, so it may be an advantage to adjust the pressure of the aqueous cooling solution or inside the cooling network before it enters the carbamate condenser such that its temperature is very close to its boiling point, for example less than 10 °C below its boiling point.

In some embodiments, the pressure at the cooling fluid outlet is controlled by a control valve. The control valve may be configured to maintain the pressure inside the cooling network close to the boiling pressure of the cooling solution directed in the cooling fluid inlet. As the temperature of the cooling solution may vary, the pressure inside the cooling network can be adapted such that the cooling solution is very close to its boiling point, thus increasing the amount of steam produced in the condenser.

In some embodiments, the fluid composition obtained in step c) is a biphasic composition comprising a liquid portion and a gas portion. The condensation of a gaseous stream comprising ammonia, carbon dioxide, and water can be done in two ways: a complete condensation or partial condensation. A complete condensation describes a method wherein all the water vapor comprised in the gaseous stream is condensed in liquid water. In such a case, the fluid composition obtained at the outlet of the carbamate condenser is a liquid. A partial condensation describes a method wherein only part of the water vapor comprised in the gaseous stream is condensed in liquid water. In such a case, the fluid composition obtained near the outlet of the carbamate condenser is a biphasic liquid, a mixture of liquid and gases. In some embodiments, the carbamate condenser comprises two fluid outlets, one to recover the liquid portion of the fluid produced by the carbamate condenser and one to recover the gas portion of the fluid produced by the carbamate condenser.

In some embodiments, the gaseous stream provided in step a) comprises from 10.0 to 50.0 weight% of ammonia. In some embodiments, the gaseous stream provided in step a) comprises from 10.0 to 40.0 weight% of carbon dioxide. The exact content in ammonia, carbon dioxide, and water in the gaseous stream provided in step a) depends on several factors, in particular the source of the gaseous stream. In some embodiments, the gaseous stream provided in step a) comprises from 10.0 to 50.0 weight% of ammonia, and from 10.0 to 40.0 weight% of carbon dioxide.

In some embodiments, the fluid composition obtained at the fluid outlet of the carbamate condenser in step c) comprises from 10.0 to 50.0 weight% of ammonia. In some embodiments, the fluid composition obtained at the fluid outlet of the carbamate condenser in step c) comprises from 10.0 to 40.0 weight% of carbon dioxide. The fluid composition obtained at the outlet of the carbamate condenser has the same chemical composition as the gaseous stream introduced in the carbamate condenser in step a) since no exchange of matter occur between the gaseous stream and the cooling fluid. The fluid obtained in step c) may be purely liquid, in which case the liquid comprises the same amount of ammonia and carbon dioxide as the gaseous stream introduced in step a). The fluid obtained in step c) may also be a mixture of liquid and gas if the condensation is only partial. In such embodiments, both the liquid phase and the gaseous phase may comprise ammonia and carbon dioxide. In some embodiments, the fluid composition obtained in step c) comprises from 10.0 to 50.0 weight% of ammonia, and from 10.0 to 40.0 weight% of carbon dioxide.

In some embodiments, the aqueous cooling solution provided in step b) comprises from 99.5 to 100 weight% of water. The aqueous cooling solution is partially or completely transformed in steam during the method, so the aqueous cooling solution should have suitable chemical properties, in particular a very low amount of impurities that could precipitate out of the solution.

In some embodiments, the aqueous cooling solution provided in step b) comprises steam condensate and/or demineralized water. A urea plant comprises many devices, including some that are configured to condense streams of pure water vapor. Such steam condensate is a pure water stream that can be used in the present method.

In some embodiments, the carbamate condenser is a shell-and-tube carbamate condenser. A shell-and-tube carbamate condenser comprises a body and a plurality of tubes configured to carry a fluid. A shell-and-tube carbamate condenser comprises two spaces where fluids can flow without mixing with one another: a tube side comprising of all the internal space of the tubes comprised inside the carbamate condenser, and the shell side comprising the volume inside the carbamate condenser that is not inside the tubes. In some embodiments, the carbamate condenser is a horizontal shell-and-tube heat exchanger, such as a kettle-type carbamate condenser. In some embodiments, the carbamate condenser is a vertical shell-and-tube carbamate condenser.

In another aspect, the present disclosure provides a method for producing a urea-containing composition, the method comprising the steps of:
a) reacting ammonia and carbon dioxide in a synthesis section, thereby producing a synthesis solution comprising urea, water, and ammonium carbamate;
b) processing the synthesis solution produced in step a), thereby producing a urea melt comprising urea and water, and one or more gaseous streams comprising ammonia, carbon dioxide, and water;
c) condensing at least one of the one or more gaseous streams in a carbamate condenser to produce steam, thereby producing a fluid composition comprising an aqueous solution comprising ammonium salts, and steam;
d) processing the urea melt produced in step b) into a urea-containing composition.
The method according to the first aspect can be integrated into a method for producing urea.

Ammonia and carbon dioxide are reacted together in the synthesis section of the plant under high pressure, for example above 10 MPa, and high temperature, for example above 150 °C. Under these conditions, two reactions occur: first ammonia and carbon dioxide form ammonium carbamate (NH₄O₂CNH₂), and secondly, ammonium carbamate is transformed into urea and water. The transformation of ammonium carbamate into urea is not complete, and an aqueous solution comprising urea, ammonium carbamate, and water, is obtained. This aqueous solution produced by the reaction of ammonia and carbon dioxide is called the synthesis solution. The synthesis solution may also comprise free ammonia.

In some embodiments, the method for producing a urea-containing composition is performed in a urea-producing plant comprising a synthesis section, a decomposition section, an evaporation section, a finishing section, and a condensation section. The synthesis section is configured to receive streams of ammonia and carbon dioxide and to produce a synthesis solution comprising urea, ammonium carbamate, free ammonia, and water. The synthesis section may comprise a reactor, a high-pressure stripper, and a high-pressure carbamate condenser. The decomposition section is configured to process the synthesis solution from the synthesis section into an aqueous urea solution comprising at least 60.0 weight% of urea and up to 40.0 weight% of water. In some embodiments, the decomposition section is configured to process the synthesis solution from the synthesis section into an aqueous urea solution comprising from 60.0 to 80.0 weight% of urea and from 20.0 to 40.0 weight% of water. The evaporation section is configured to process the aqueous urea solution from the decomposition section into a urea melt comprising at least 95.0 weight%, or from 95.0 to 99.9 weight%, of urea and water. The finishing section is configured to transform a urea melt comprising at least 95.0 weight% of urea into a finished urea composition, which may be solid, such as urea prills, or urea granules, or liquid, such as an aqueous urea solution comprising from 30.0 to 35.0 weight% of urea, for example a diesel exhaust fluid. The condensation section comprises a condenser that is configured to perform the method described above and to generate steam from the condensation of a gaseous stream comprising ammonia and carbon dioxide.

In some embodiments, the urea-producing plant comprises a hydrolyzer section, also called a wastewater section. The hydrolyzer section is configured to receive streams comprising urea, in particular a low amount, for example less than 10.0 weight%, of urea, and to decompose urea to ammonia and carbon dioxide. The hydrolyzer section may be configured to produce gaseous streams comprising ammonia and carbon dioxide. The hydrolyzer section may be connected to the condensation section of the urea-producing plant, such that the gaseous streams produced therein may be condensed into liquid streams and recycled back to the synthesis plant.

In some embodiments, the synthesis solution comprises from 40.0 to 60.0 weight% of urea, from 10.0 to 25.0 weight% of ammonium carbamate, from 0.0 to 20.0 weight% of free ammonia, and from 20.0 to 30.0 weight% of water.

To produce a urea-based composition, the synthesis solution needs to be purified and concentrated to obtain a urea melt. A urea melt is a composition comprising urea, in particular at least 90.0 weight% urea or from 90.0 to 100 weight% of urea, and from 0.1 to 5.0 weight% of water.

To obtain a urea melt, the synthesis solution is processed in different devices, such as carbamate decomposer, and evaporators. Some of these devices produce a gaseous stream comprising ammonia, carbon dioxide, and water. A carbamate decomposer is a device configured to receive an aqueous solution comprising urea, ammonium carbamate, water, and optionally free ammonia. A carbamate decomposer is a heat exchanger wherein the aqueous solution is heated up, for example with steam, to decompose the ammonium carbamate into ammonia and carbon dioxide. The gaseous ammonia and carbon dioxide are separated from the liquid phase either within the carbamate condenser, or in a liquid/vapor separator located downstream of the carbamate decomposer. A carbamate decomposer produces an aqueous urea solution depleted of ammonium carbamate. The aqueous urea solution produced by a carbamate decomposer may still comprise ammonium carbamate.

In some embodiments, the processing step b) comprises directing the synthesis solution in one or two carbamate decomposers.

In some embodiments, the processing step b) comprises directing the synthesis solution in one or two carbamate decomposers, thereby producing an aqueous urea solution comprising less than 5.0 weight% of ammonium carbamate, and optionally free ammonia.

In some embodiments, the processing step b) comprises directing the synthesis solution in one or two carbamate decomposers, thereby producing an aqueous urea solution that consists of urea and water.

In some embodiments, the processing step b) comprises the steps of processing the synthesis solution into an aqueous urea solution comprising urea, water, and less than 5.0 weight% of components other than urea and water, and concentrating this aqueous urea solution to a urea melt.

Concentrating an aqueous urea solution into a urea melt may be done in evaporators. An evaporator is a device configured to receive an aqueous urea solution and heat up this aqueous urea solution to remove water and remaining ammonium carbamate and free ammonia from it. The heat source may be steam.

To increase the yield of the plant, it is important to recycle the starting materials, ammonia and carbon dioxide, to the synthesis section. However, it is preferred to re-inject these starting materials in a liquid form, not in a gaseous form. So, these gaseous streams are condensed into a liquid aqueous solution in a carbamate condenser according to the method described. In addition to produce a liquid stream that can be re-injected in the synthesis section, this condensation produces heat that can be used to generate steam that can also be re-used in the urea plant, thereby lowering the requirement of steam production, which is often done by burning fossil fuels, which generate carbon dioxide emissions, a greenhouse gas.

In some embodiments, the method comprises compressing the steam produced in step c) to a pressure ranging from 0.3 to 2.5 MPa. The steam produced in the carbamate condenser may have a pressure that is too low to be re-used as such in the urea plant. Many devices in a urea plant require steam having a pressure of at least 300 kPa. So, to re-use the steam produced by the carbamate condenser may require to first increase the pressure of that steam, for example using a gas compressor, to obtain steam that can be directed to a device in the urea plant.

In another aspect, the present disclosure provides a urea-producing plant for producing a urea-based composition comprising:
- a synthesis section configured to produce an aqueous urea solution comprising urea, water, and ammonium carbamate;
- a decomposition section connected to the synthesis section, the decomposition section being configured to receive an aqueous urea solution, and transform said aqueous urea solution into a pure urea solution comprising urea, water, and less than 5.0 weight% of impurities;
- an evaporation section connected to the decomposition section configured to remove water from the pure urea solution comprising urea, water, and less than 5.0 weight% of impurities to obtain a urea melt comprising urea and less than 5.0 weight% of water;
- a finishing section configured to receive a urea melt and produce a urea-containing product;
- a condensation section connected to the decomposition section and the evaporation section, wherein:
   - the condensation section is configured to transform gaseous streams comprising ammonia, carbon dioxide and water, from the decomposition section and/or the evaporation section, into an aqueous solution comprising ammonium salts;
   - the condensation section is also connected to the synthesis section and configured to send the aqueous solution comprising ammonium salts to the synthesis section; and
   - the condensation section comprises at least one carbamate condenser configured to produce low-pressure steam from the condensation of a gaseous stream comprising ammonia, carbon dioxide, and water.

In some embodiments, the condensation section is configured to process a gaseous stream comprising ammonia, carbon dioxide, and water, having a temperature ranging from 100 to 140 °C and a pressure ranging from 0.1 to 4.0 MPa.

In some embodiments, the synthesis section comprises a reactor, a high-pressure stripper, and a high-pressure carbamate condenser.

In some embodiments, the decomposition section comprises one or more heat exchangers configured to decompose ammonium carbamate comprised in an aqueous solution. In some embodiments, the decomposition section comprises one or more liquid-vapor separators.

In some embodiments, the evaporation section comprises one or more evaporators, such as two evaporators. The evaporators are configured to remove water from an aqueous solution comprising urea.

In some embodiments, the finishing section comprises a granulator, such as a fluidized bed granulator, or a prilling tower. In some embodiments, the finishing sections comprises a mixing device for adding one or more components, such as a urease inhibitor (for example N-n-butylthiophosphoric amide), a nitrification inhibitor (for example 3,4-dimethylpyrazole phosphate), a granulation additive (for example urea formaldehyde), a macro nutrient (nitrogen, phosphorus, potassium), a secondary nutrient (magnesium, calcium, sulfur), or a micro nutrient (zinc, copper, boron, manganese, molybdenum, iron), to a urea melt.

In some embodiments, the finishing section comprises a scrubber for removing pollutants, such as ammonia and urea, from a gaseous stream.

In some embodiments, the urea-producing plant comprises a hydrolyzer section connected to the evaporations section and to the condensation section, the hydrolyzer section being configured to remove urea from liquid aqueous streams urea and send a gaseous stream comprising water, ammonia, and carbon dioxide to the condensation section.

In another aspect, the present disclosure provides the use of a carbamate condenser to produce steam, wherein the carbamate condenser is configured to receive a gaseous stream comprising ammonia, carbon dioxide, and water to the fluid inlet, the gaseous stream having a temperature ranging from 100 to 140 °C and a pressure ranging from 0.1 to 4.0 MPa.

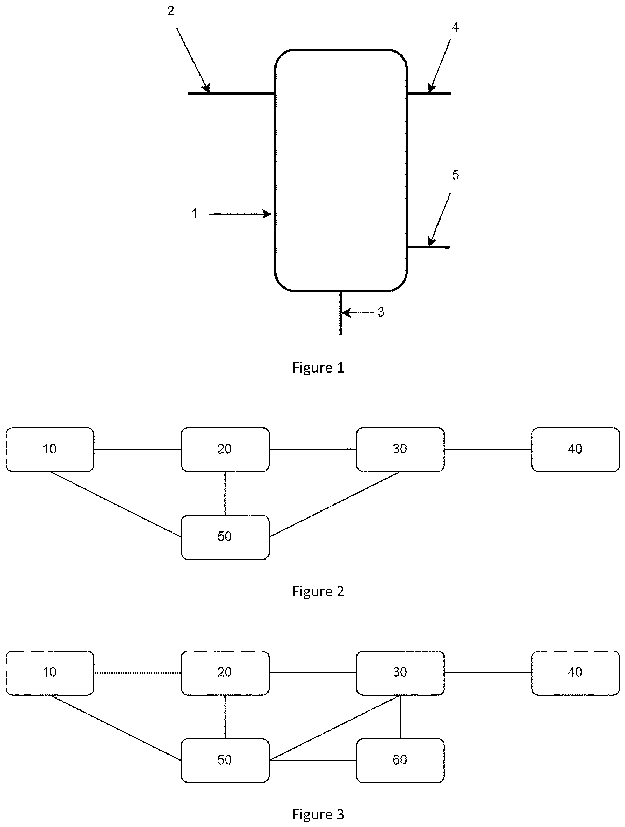

Figure 1 shows a carbamate condenser **1** suitable for performing the method according to the first aspect of the present disclosure. The carbamate condenser **1** is a vertical shell-and-tube heat exchanger comprising a fluid inlet **2,** a fluid outlet **3,** an inlet for a cooling fluid **5,** and an outlet for a cooling fluid **4.** The fluid inlet **2** is located near the top of the condenser **1,** such that the condensate generated inside the carbamate condenser **1** flows naturally towards the fluid outlet **3** by gravity. The carbamate condenser **1** comprises a plurality of tubes not shown on the figure. These tubes can be fluidly connected to the inlet for a cooling fluid **5** and the outlet for a cooling fluid **4,** such that the cooling fluid flows through the tubes. Alternatively, the tubes can be fluidly connected to the fluid inlet **2** and the fluid outlet **3,** such that the gas stream and condensate obtained from the gaseous stream flow in the tubes.

Figure 2 shows a schematic representation of a urea-producing plant suitable for producing urea according to a method disclosed herein. The urea-producing plant comprises a synthesis section **10,** configured to produce an aqueous urea solution comprising urea, water, and ammonium carbamate. The synthesis section **10** is configured to receive a stream of gaseous ammonia, gaseous carbon dioxide, and high-pressure steam. The synthesis section **10** is connected to a decomposition section **20** configured to receive an aqueous urea solution, and transform said aqueous urea solution into a pure urea solution comprising urea, water, and less than 5.0 weight% of impurities. In addition to the pure urea solution, the decomposition section **20** is also configured to produce one or more gaseous streams comprising ammonia, carbon dioxide, and water. The decomposition section **20** is connected to an evaporation section **30** and a condensation section **50.** The evaporation section **30** is configured to receive the pure urea solution from the decomposition section **20** and remove water from said pure urea solution to obtain a urea melt comprising urea and less than 5.0 weight% of water. The evaporation section **30** is connected to a finishing section **40** that is configured to receive a urea melt and produce a urea-containing product. The urea-containing product may be a solid product, such as granules or prills, or a liquid product, such as a urea solution, or a urea ammonium nitrate solution. The condensation section **50** is configured to receive gaseous streams from the decomposition section **20** and the evaporation section **30,** the gaseous streams comprising ammonia, carbon dioxide and water, to transform said gaseous streams into an aqueous solution comprising ammonium salts, such as ammonium carbamate. The condensation section **50** is connected to the synthesis section **10** and is configured to send the aqueous solution comprising ammonium salts to the synthesis section **10.** The condensation section **50** comprises at least one carbamate condenser configured to produce low-pressure steam from the condensation of a gaseous stream comprising ammonia, carbon dioxide, and water. The low-pressure steam produced by the condensation section **50** can be sent to the synthesis section **10,** the decomposition section **20,** and/or the evaporation section **30,** depending on the steam balance of the plant and the requirements of the different sections. The low-pressure steam can be compressed to a higher pressure depending on the section of the plant it is sent to.

Figure 3 shows a schematic representation of another urea-producing plant suitable for producing urea according to a method disclosed herein. The urea-producing plant comprises the same sections as the plant shown in figure 2: a synthesis section **10,** a decomposition section **20,** an evaporation section **30,** a finishing section **40,** and a condensation section **50.** The urea-producing plant further comprises a hydrolyzer section **60,** configured to treat liquid streams comprising urea; such a section may be called a wastewater section. Some sections of the plant, such as the evaporation section **30,** may produce waste streams, liquid or gaseous, comprising small amounts of urea. The gaseous streams may be condensed into a liquid stream comprising urea in the evaporation section **30** or the hydrolyzer section **60.** In the hydrolyzer section **60,** the liquid streams comprising urea are heated up, such that urea decomposes back into ammonia and carbon dioxide in gaseous phase. The gas streams are directed to the condensation section **50,** to be recycled back to the synthesis section **10.** The condensation section **50** may be configured to combine the gaseous streams of the condensation section **30** and the hydrolyzer section **60** prior to their condensation.

## Claims

1. A method for producing steam in a carbamate condenser, the carbamate condenser comprising a fluid inlet, a fluid outlet, a cooling fluid inlet, and a cooling fluid outlet, the method comprising the steps of:
a) providing a gaseous stream comprising ammonia, carbon dioxide, and water to the fluid inlet, wherein the gaseous stream has a temperature ranging from 100 to 140 °C and a pressure ranging from 0.1 to 4.0 MPa;
b) providing an aqueous cooling solution to the cooling fluid inlet, wherein the temperature of the aqueous cooling solution ranges from 60 to 110 °C;
c) obtaining a fluid composition from the fluid outlet, the fluid composition comprising an aqueous solution comprising ammonium salts;
d) obtaining steam from the cooling fluid outlet.

2. The method according to claim 1, wherein the steam obtained from the cooling fluid outlet has a pressure ranging from 20 to 150 kPa.

3. The method according to any one of claims 1 to 2, wherein the aqueous cooling solution provided in step b) has a pressure ranging from 20 to 150 kPa.

4. The method according to any one of claims 1 to 3, wherein the fluid composition obtained in step c) is a biphasic composition comprising a liquid portion and a gas portion.

5. The method according to any one of claims 1 to 4, wherein the gaseous stream provided in step a) comprises from 10.0 to 50.0 weight% of ammonia.

6. The method according to any one of claims 1 to 5, wherein the gaseous stream provided in step a) comprises from 10.0 to 40.0 weight% of carbon dioxide.

7. The method according to any one of claims 1 to 6, wherein the aqueous cooling solution provided in step b) comprises from 99.5 to 100 weight% of water.

8. The method according to any one of claims 1 to 7, wherein the aqueous cooling solution provided in step b) comprises steam condensate and/or demineralized water.

9. A method for producing a urea-containing composition, the method comprising the steps of:
a) reacting ammonia and carbon dioxide in a synthesis reactor, thereby producing a synthesis solution comprising urea, water, and ammonium carbamate;
b) processing the synthesis solution produced in step a), thereby producing a urea melt comprising urea and water, and one or more gaseous streams comprising ammonia, carbon dioxide, and water;
c) condensing at least one of the one or more gaseous streams in a condenser to produce steam, thereby producing a fluid composition comprising an aqueous solution comprising ammonium salts, and steam;
d) processing the urea melt produced in step b) into a urea-containing composition.

10. The method according to claim 9, wherein the method comprises the further step:
e) compressing the steam produced in step c) to a pressure ranging from 0.3 to 2.5 MPa.

11. A urea-producing plant for producing a urea-based composition comprising:
- a synthesis section (10) configured to produce an aqueous urea solution comprising urea, water, and ammonium carbamate;
- a decomposition section (20) connected to the synthesis section (10), the decomposition section (20) being configured to receive an aqueous urea solution, and transform said aqueous urea solution into a pure urea solution comprising urea, water, and less than 5.0 weight% of impurities;
- an evaporation section (30) connected to the decomposition section (20) configured to remove water from the pure urea solution comprising urea, water, and less than 5.0 weight% of impurities to obtain a urea melt comprising urea and less than 5.0 weight% of water;
- a finishing section (40) configured to receive a urea melt and produce a urea-containing product;
- a condensation section (50) connected to the decomposition section (20) and the evaporation section (30), wherein:
∘ the condensation section (50) is configured to transform gaseous streams comprising ammonia, carbon dioxide and water, from the decomposition section (20) and/or the evaporation section (30), into an aqueous solution comprising ammonium salts;
∘ the condensation section (50) is also connected to the synthesis section (10) and configured to send the aqueous solution comprising ammonium salts to the synthesis section (10);
∘ and the condensation section (50) comprises at least one carbamate condenser configured to produce low-pressure steam from the condensation of a gaseous stream comprising ammonia, carbon dioxide, and water.

12. The urea-producing plant according to claim 11, further comprising a hydrolyzer section (60) connected to the evaporations section (30) and to the condensation section (50), the hydrolyzer section (60) being configured to remove urea from liquid aqueous streams urea and send a gaseous stream comprising water, ammonia, and carbon dioxide to the condensation section (50).

13. Use of a carbamate condenser to produce steam, wherein the carbamate condenser receives a gaseous stream comprising ammonia, carbon dioxide, and water to the fluid inlet, the gaseous stream having a temperature ranging from 100 to 140 °C and a pressure ranging from 0.1 to 4.0 MPa.

14. A urea-producing plant for producing a urea-based composition comprising:
- a synthesis section configured to produce an aqueous urea solution comprising urea, water, and ammonium carbamate;
- a decomposition section connected to the synthesis section, the decomposition section being configured to receive an aqueous urea solution, and transform said aqueous urea solution into a pure urea solution comprising urea, water, and less than 5.0 weight% of impurities;
- an evaporation section connected to the decomposition section configured to remove water from the pure urea solution comprising urea, water, and less than 5.0 weight% of impurities to obtain a urea melt comprising urea and less than 5.0 weight% of water;
- a finishing section configured to receive a urea melt and produce a urea-containing product;
- a condensation section connected to the decomposition section and the evaporation section, wherein:
- the condensation section is configured to transform gaseous streams comprising ammonia, carbon dioxide and water, from the decomposition section and/or the evaporation section, into an aqueous solution comprising ammonium salts;
- the condensation section is also connected to the synthesis section and configured to send the aqueous solution comprising ammonium salts to the synthesis section; and
- the condensation section comprises at least one carbamate condenser configured to produce low-pressure steam from the condensation of a gaseous stream comprising ammonia, carbon dioxide, and water.
